# EUROPEAN PATENT APPLICATION

(11) **EP 2 008 677 A1**
(43) Date of publication of application: **31.12.2008**
(21) Application number: 07702222.6
(22) Date of filing: 29.01.2007
(51) Int. Cl.: A61M 5/14, A61J 1/14

(54) **RIGID DOUBLE-PORT TUBE WITH MEDICINE DISPENSING NOZZLE FOR THE BIG TRANSFUSION SOFT BAG**

(30) Priority: 19.04.2006 CN 200620050657 U
(71) Applicant: Hunan Chinasun Pharmaceutical Machinery Co, Ltd, Hunan 410100 (CN)
(72) Inventor: LIU, Xianghua, Changsha Hunan 410100 (CN); PENG, Xunde, Changsha Hunan 410100 (CN)
(74) Representative: Shaw, Matthew Nigel
(86) International application number: PCT/CN2007/000327
(87) International publication number: WO 2007/118393

(57) **Abstract**

A rigid double-port tube with medicine dispensing nozzle for the big transfusion soft bag, which consists of a rigid double-port (1) and a medicine dispensing nozzle (2) through which the medicine is dispensed, through the rigid double-port (1) the medicine is infused into the soft bag and the medicine liquid is exported from the soft bag; the rigid double-port (1) consists of a medicine infusing port (11), a medicine dispensing port (12) disposed parallel to the medicine infusing port (11), and the connecting base (13) connecting the medicine infusing port (11) and the medicine dispensing port (12), the medicine dispensing port (12) is connected with the medicine dispensing nozzle (2) hermetically; a sealing rubber plug (22) used to medicine dispensing is disposed onto the medicine dispensing nozzle (2), the sealing rubber plug (22) can be pierced by the syringe pinhead and it contracts and closes automatically after the syringe pinhead being pulled out.

## Description

### Technical Field

The present invention belongs to the field of pharmaceutical packaging material, in particular relates to a port for big transfusion soft bag packaging.

### Background Art

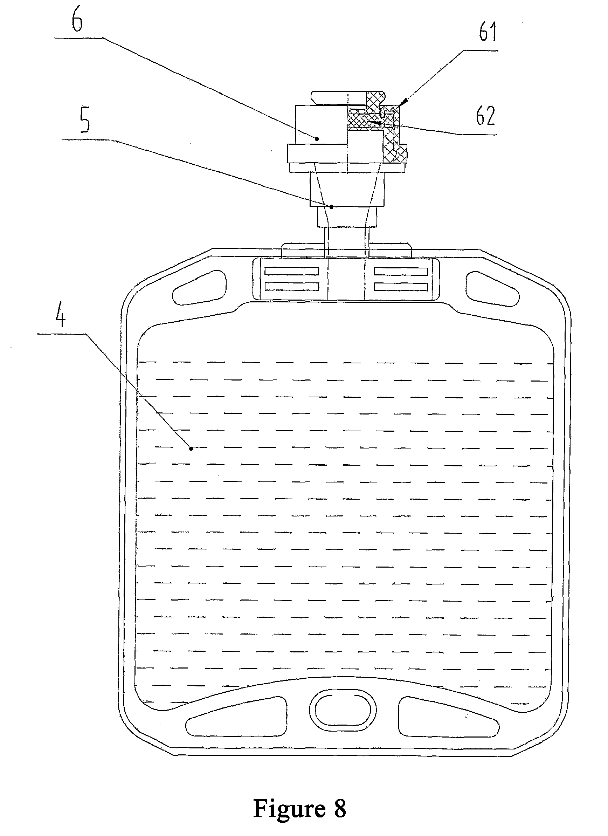

As for the current rigid port for big transfusion soft bag, both medicine dispensation and transfusion are through a same rubber plug 62 of combining lid of a lift-ring type combining lid 6 (as shown in figure 8), the rubber plug of combining lid 62 is exposed in the air and is contaminated, as time and place for dispensation and medication are different, it is easy for the pinhead of transfusion apparatus to bring the pathogen attached on the rubber plug of combining lid 62 into the medicine liquid during transfusion so that it is easy to cause safety accident of transfusion.

### Summary of the Invention

In order to overcome the defects of the above prior art, the present invention aims to solve the technical problem and provide a rigid double-port tube with medicine dispensing nozzle for the big transfusion soft bag, which separates the medicine dispensing port from the transfusion port so as to facilitate and secure the medicine dispensation and transfusion and prevent the medicine liquid from being contaminated, through which the medicine can be directly infused into the bag for loading medicine, and which has simple structure and production process and low production cost.

In order to solve the above technical problem, the present invention adopts the following technical solution: a rigid double-port tube with medicine dispensing nozzle for the big transfusion soft bag according to the present invention is characterized in that it comprises a rigid double-port and a medicine dispensing nozzle through which the medicine is dispensed, through the rigid double-port the medicine is infused into the soft bag and the medicine liquid is exported from the soft bag; the rigid double-port comprises a medicine infusing port, a medicine dispensing port disposed parallel to the medicine infusing port, and a connecting base connecting the two ports as a whole, the medicine dispensing port is connected with the medicine dispensing nozzle hermetically; a sealing rubber plug used to medicine dispensing is disposed onto the medicine dispensing nozzle, the sealing rubber plug can be pierced by the syringe pinhead and it contracts and closes automatically after the syringe pinhead being pulled out.

As a further improvement of the present invention, the medicine infusing port comprises a medicine infusing channel and a medicine infusing port connecting ring disposed at the upper end of the medicine infusing channel, the medicine dispensing port comprises a medicine dispensing channel and a medicine dispensing port connecting ring disposed at the upper end of the medicine dispensing channel, and the medicine dispensing port connecting ring is connected with the medicine dispensing nozzle by welding or pressing hermetically.

As a further improvement of the present invention, the medicine dispensing nozzle provided with a rubber plug comprises an outer lid, inside which the rubber plug is provided and at the top of which an easy-breaking cap is provided, the rubber plug being exposed after breaking the easy-breaking cap, and the outer lid is connected with the medicine dispensing port connecting ring provided for the medicine dispensing port by welding or pressing hermetically. Furthermore, the lower portion of the outer lid can be covered outside the medicine dispensing port, a pressing depression is provided at the connecting portion between the outer lid and the medicine dispensing port, a pressing protrusion is provided at the corresponding place on the medicine dispensing port connecting ring of the medicine dispensing port, the outer lid is connected with the medicine dispensing port connecting ring by pressing hermetically.

A sealing film can be provided at the upper end of the inner cavity of the medicine dispensing channel. The sealing film can prevent the medicine liquid from contacting the rubber plug of the medicine dispensing nozzle and it is easy to be pierced by the syringe pinhead during medicine dispensing at the same time.

As another further improvement of the present invention, the medicine dispensing nozzle provided with a rubber plug comprises a connecting lid and an outer lid that is connected with the upper portion of the connecting lid. A lid sealing film that can be pierced by a needle cylinder is provided in the inner cavity of the connecting lid. The rubber plug is sealed between the lid sealing film of the connecting lid and the outer lid. An easy-breaking cap is provided at the top of the outer lid. The rubber plug can be exposed after breaking of the easy-breaking cap. The lower portion of the connecting lid is connected with the medicine dispensing port by welding hermetically. A lid connecting ring, which is connected with the medicine dispensing port connecting ring provided for the medicine dispensing port by welding hermetically, is provided at the bottom of the connecting lid. The upper portion of the connecting lid is connected with the outer lid by welding or pressing hermetically.

The welded surface on the connecting base welded to the bag film of the big transfusion soft bag is a lip-like welded surface easy for welding.

Combining the present invention with the big transfusion soft bag, the medicine can be dispensed through medicine dispensing port and the transfusion can be performed through the medicine infusing port, thereby the problem that dispensation and transfusion use one channel together is solved, such that dispensation and transfusion become easy and safe and the medicine liquid is prevented from being contaminated. The structure thereof is simple and cost is low. In the present invention, the medicine can be infused to the bag for loading medicine of the transfusion bag directly via the infusing channel, such that the production process of the big transfusion soft bag packaging of the rigid double-port tube is similar to that of the original big transfusion soft bag packaging of the rigid single-port tube, the production equipment is relative simple, the production process is simple and the production cost is low. The medicine dispensing nozzle of the present invention comprises the outer lid and the rubber plug or comprises the outer lid, the rubber plug and the connecting lid. Moreover, the easy-breaking cap on the outer lid is used to replace the ring tab on the outer lid in prior art such that the price of the raw material is low. Also, the cost can be saved since the structure of an inner rubber plug is simpler than the original structure. In addition, the structure of the whole medicine dispensing nozzle is simple and the cost thereof is greatly reduced.

### Brief Description Of The Drawings

Figure 1 is a structural schematic front view of the first embodiment of the present invention;
Figure 2 is a structural schematic bottom view of the first embodiment of the present invention;
Figure 3 is a structural schematic front view of the second embodiment of the present invention;
Figure 4 is a structural schematic bottom view of the second embodiment of the present invention;
Figure 5 is a structural schematic front view of the third embodiment of the present invention; and
Figure 6 is a structural schematic bottom view of the third embodiment of the present invention.
Figure 7 is a structural schematic view used in the big transfusion soft bag packaging according to the first embodiment of the present invention;
Figure 8 is a structural schematic view for the single-port tube of the prior art used in the big transfusion soft bag packaging.

The reference signs in the figures represent:
1. rigid double-port 11. medicine infusing port
111. medicine infusing port connecting ring
1111. medicine infusing port pressing protrusion
112. medicine infusing channel 12. medicine dispensing port
121. medicine dispensing port connecting ring 1211. pressing protrusion
122. medicine dispensing channel 1221. sealing film
13. connecting base 131. lip-like welded surface
2. medicine dispensing nozzle 21. outer lid
211. easy-breaking cap 212. pressing depression
22. rubber plug 23. connecting lid
231. lid sealing film 3. combining lid
4. bag for loading medicine 5. rigid single-port tube
6. lift-ring combining lid 61. outer lid of combining lid
62. rubber plug of combining lid.

### Description Of Preferred Embodiments

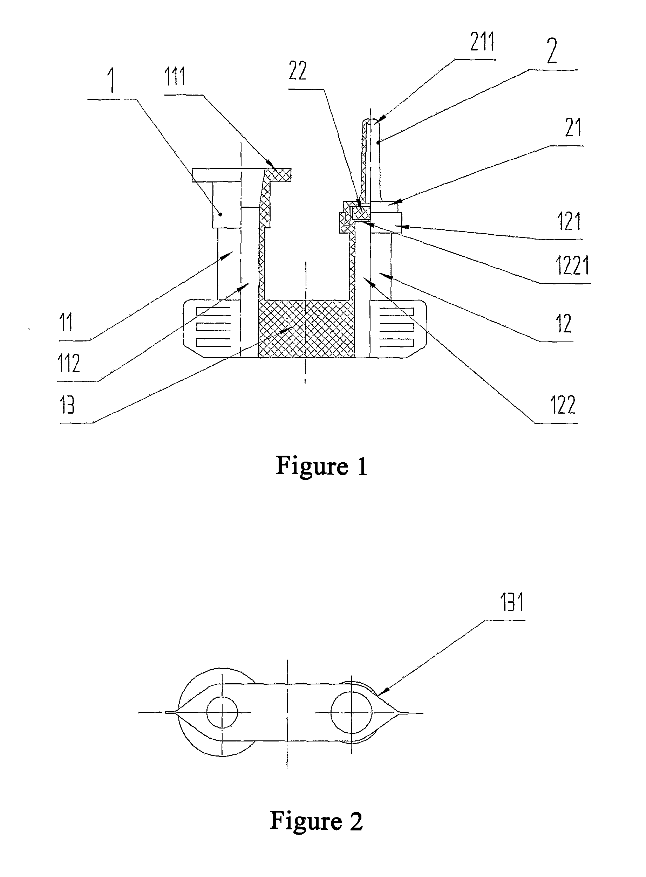

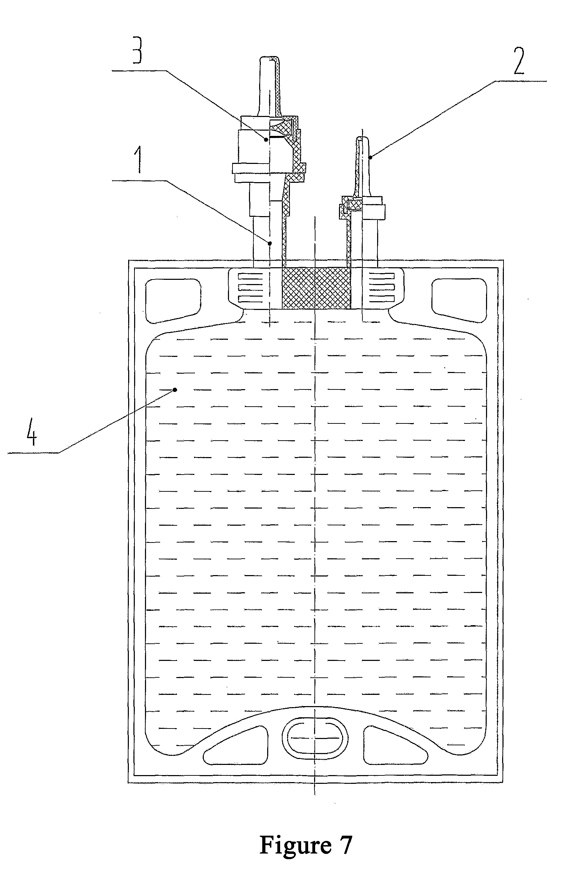

Figures 1 and 2 show the first embodiment of the present invention. A rigid double-port tube with medicine dispensing nozzle for the big transfusion soft bag according to the present invention comprises a rigid double-port 1 and a medicine dispensing nozzle 2 through which the medicine is dispensed, through the rigid double-port 1 the medicine is infused into the soft bag and the medicine liquid is exported from the soft bag; the rigid double-port 1 comprises a medicine infusing port 11, a medicine dispensing port 12 disposed parallel to the medicine infusing port 11, and a connecting base 13 connecting the two ports as a whole, the medicine dispensing port 12 is connected with the medicine dispensing nozzle 2 hermetically; a sealing rubber plug 22 used to medicine dispensing is disposed onto the medicine dispensing nozzle 2, the sealing rubber plug 22 can be pierced by the syringe pinhead and it contracts and closes automatically after the syringe pinhead being pulled out. The medicine infusing port 11 comprises a medicine infusing channel 112 and a medicine infusing port connecting ring 111 disposed at the upper end of the medicine infusing channel 112. The medicine dispensing port 12 comprises a medicine dispensing channel 122 and a medicine dispensing port connecting ring 121 disposed at the upper end of the medicine dispensing channel 122, and the medicine dispensing port connecting ring 121 is connected with the medicine dispensing nozzle 2 by welding or pressing hermetically. The welded surface between the connecting base 13 and the bag film of the bag for loading medicine 4 (see figure 7) is a lip-like welded surface 131 easy for welding, or welded surface in other shapes, which can be welded to the bag film stably and reliably. The medicine dispensing nozzle 2 provided with a rubber plug 22 comprises an outer lid 21, inside which the rubber plug 22 is provided. A sealing film 1221 is provided at the upper end of the inner cavity of the medicine dispensing channel 122, which can prevent the medicine liquid from contacting the rubber plug 22 of the medicine dispensing nozzle 2 and it is easy to be pierced by the syringe pinhead during medicine dispensing at the same time. The rubber plug 22 is disposed between the outer lid 21 and the sealing film 1221. An easy-breaking cap 211 is provided at the top of the outer lid 21. The rubber plug 22 can be exposed after breaking of the easy-breaking cap 211, at the bottom of which a breaking crease can be disposed. The easy-breaking cap 211 can be easily broken in use to expose the rubber plug 22, i.e. the medicine can be dispensed through the medicine dispensing port 12. The easy-breaking cap 211 also can be other forms easy to be broken except for the forms shown in figures. The bottom of the outer lid 21 is connected with the medicine dispensing port connecting ring 121 provided for the medicine dispensing port 12 by welding hermetically.

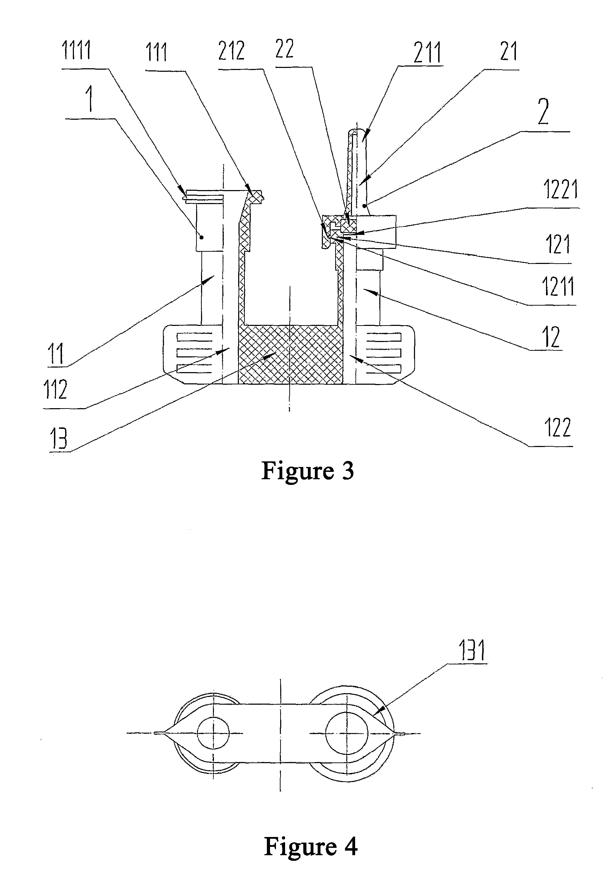

Figures 3 and 4 show the second embodiment of the present invention. The differences between this embodiment and the first embodiment lie in that the lower portion of the outer lid 21 is covered outside the medicine dispensing port 12, and the outer lid 21 is connected with the medicine dispensing port connecting ring 121 by pressing hermetically. In order to connect the outer lid 21 to the medicine dispensing port 12 closely, a pressing depression 212 is provided at the connecting portion between the outer lid 21 and the medicine dispensing port 12, and a pressing protrusion 1211 is provided at the corresponding place on the medicine dispensing port connecting ring 121 of the medicine dispensing port 12, furthermore a medicine infusing port pressing protrusion 1111 is provided on the medicine infusing port connecting ring 111.

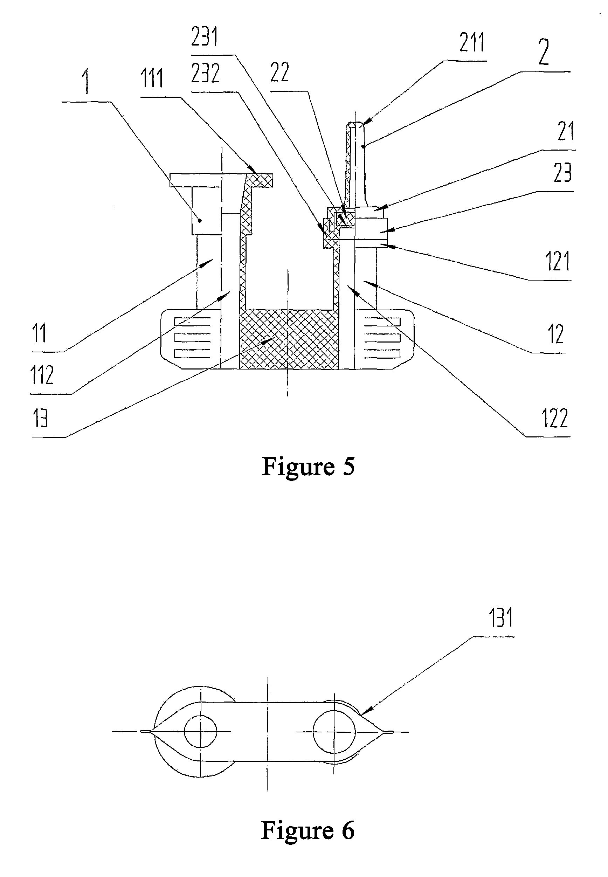

Figures 5 and 6 show the third embodiment of the present invention. The present embodiment consists of a rigid double-port 1 and a medicine dispensing nozzle 2 through which the medicine is dispensed, wherein through the rigid double-port 1 the medicine is infused into the soft bag and the medicine liquid is exported from the soft bag. The rigid double-port 1 consists of a medicine infusing port 11, a medicine dispensing port 12 disposed parallel to the medicine infusing port 11, and a connecting base 13 connecting the two ports as a whole. The medicine dispensing port 12 is connected with the medicine dispensing nozzle 2 hermetically; a sealing rubber plug 22 used to medicine dispensing is disposed onto the medicine dispensing nozzle 2. The sealing rubber plug 22 can be pierced by the syringe pinhead and it contracts and closes automatically after the syringe pinhead being pulled out. The medicine infusing port 11 consists of a medicine infusing channel 112 and a medicine infusing port connecting ring 111 disposed at the upper end of the medicine infusing channel 112. The medicine dispensing port 12 consists of a medicine dispensing channel 122 and a medicine dispensing port connecting ring 121 disposed at the upper end of the medicine dispensing channel 122, and the medicine dispensing port connecting ring 121 is connected with the medicine dispensing nozzle 2 by welding hermetically. The welded surface between the connecting base 13 and the bag film of the big transfusion soft bag is a lip-like welded surface 131 which is easy for welding, or a welded surface in other shapes, which can be welded to the bag film stably and reliably. The medicine dispensing nozzle 2 provided with a rubber plug 22 comprises a connecting lid 23 and an outer lid 21 that is connected with the upper portion of the connecting lid 23 by welding or pressing hermetically. A lid sealing film 231 that can be pierced by the pinhead is provided in the inner cavity of the connecting lid 23. The rubber plug 22 is sealed between the lid sealing film 231 of the connecting lid 23 and the outer lid 21. An easy-breaking cap 211 is provided at the top of the outer lid 21, and the rubber plug 22 can be exposed after breaking the easy-breaking cap 211. A lid connecting ring 232, which is connected with the medicine dispensing port connecting ring 121 provided for the medicine dispensing port 12 by welding hermetically, is provided at the bottom of the connecting lid 23.

Figure 7 is a structural schematic view used in the big transfusion soft bag packaging according to the first embodiment of the present invention. In the present invention, the connecting base 13 is connected to the bag for loading medicine 4 by welding and the medicine infusing port 11 is sealed by the combining lid 3.

## Claims

1. A rigid double-port tube with medicine dispensing nozzle for the big transfusion soft bag, **characterized in that** the rigid double-port tube comprising a rigid double-port (1) and a medicine dispensing nozzle (2) through which the medicine is dispensed, through the rigid double-port (1) the medicine being infused into the soft bag and the medicine liquid being exported from the soft bag; the rigid double-port (1) comprising a medicine infusing port (11), a medicine dispensing port (12) disposed parallel to the medicine infusing port (11), and a connecting base (13) connecting the two ports as a whole, the medicine dispensing port (12) being connected with the medicine dispensing nozzle (2) hermetically; a sealing rubber plug (22) used to medicine dispensing being disposed onto the medicine dispensing nozzle (2), the sealing rubber plug (22) being pierced by the syringe pinhead and it contracting and closing automatically after the syringe pinhead being pulled out.

2. The rigid double-port tube with medicine dispensing nozzle for the big transfusion soft bag according to Claim 1, **characterized in that** the medicine infusing port (11) comprising a medicine infusing channel (112) and a medicine infusing port connecting ring (111) disposed at an upper end of the medicine infusing channel (112); the medicine dispensing port (12) comprising a medicine dispensing channel (122) and a medicine dispensing port connecting ring (121) disposed at an upper end of the medicine dispensing channel (122); and the medicine dispensing port connecting ring (121) being connected with the medicine dispensing nozzle (2) by welding or pressing hermetically.

3. The rigid double-port tube with medicine dispensing nozzle for the big transfusion soft bag according to Claim 2, **characterized in that** the medicine dispensing nozzle (2) provided with a rubber plug (22) comprises an outer lid (21), inside which the rubber plug (22) is provided, an easy-breaking cap (211) being provided at the top of the outer lid (21), the rubber plug (22) being exposed after breaking of the easy-breaking cap (211), the outer lid (21) being connected with the medicine dispensing port connecting ring (121) for the medicine dispensing port (12) by welding or pressing hermetically.

4. The rigid double-port tube with medicine dispensing nozzle for the big transfusion soft bag according to Claim 3, **characterized in that** a lower portion of the outer lid (21) is covered outside the medicine dispensing port (12), a pressing depression (212) being provided at the connecting portion between the outer lid (21) and the medicine dispensing port (12), a pressing protrusion (1211) being provided at a corresponding place on the medicine dispensing port connecting ring (121) of the medicine dispensing port (12), the outer lid (21) being connected with the medicine dispensing port connecting ring (121) by pressing hermetically.

5. The rigid double-port tube with medicine dispensing nozzle for the big transfusion soft bag according to Claim 2, or 3 or 4, **characterized in that** a sealing film (1221) is provided at an upper end of inner cavity of a medicine dispensing channel (122), wherein the sealing film (1221) can prevent medicine liquid from contacting the rubber plug (22) of the medicine dispensing nozzle (2) and is easy to be pierced by the syringe pinhead during medicine dispensing at the same time.

6. The rigid double-port tube with medicine dispensing nozzle for the big transfusion soft bag according to Claim 2, **characterized in that** the medicine dispensing nozzle (2) provided with a rubber plug (22) comprises a connecting lid (23) and an outer lid (21) that is connected with an upper portion of the connecting lid (23), a lid sealing film (231) that can be pierced by the pinhead being provided in inner cavity of the connecting lid (23), the rubber plug (22) being sealed between the lid sealing film (231) of the connecting lid (23) and the outer lid (21), an easy-breaking cap (211) being provided at top of the outer lid (21), the rubber plug (22) being exposed after breaking the easy-breaking cap (211), a lower portion of the connecting lid (23) being connected with the medicine dispensing port (12) by welding hermetically.

7. The rigid double-port tube with medicine dispensing nozzle for the big transfusion soft bag according to Claim 6, **characterized in that** a lid connecting ring (232) is provided at the bottom of the connecting lid (23), which is connected with the medicine dispensing port connecting ring (121) provided for the medicine dispensing port (12) by welding hermetically, the upper portion of the connecting lid (23) being connected with the outer lid (21) by welding or pressing hermetically.

8. The rigid double-port tube with medicine dispensing nozzle for the big transfusion soft bag according to Claims 1, 2, 3, 4, 6 or 7, **characterized in that** a welded surface on the connecting base (13) welded to a bag film of the big transfusion soft bag is a lip-like welded surface (131) easy for welding.
